# EUROPEAN PATENT SPECIFICATION

(11) **EP 3 962 408 B1**
(45) Date of publication and mention of the grant of the patent: **21.06.2023**
(21) Application number: 20731549.0
(22) Date of filing: 30.04.2020
(51) Int. Cl.: A61C 19/045

(54) **AN APPARATUS FOR TRACKING AND RECORDING THE MOVEMENTS OF A PERSON'S JAW AND THE RELATIVE METHOD**
VORRICHTUNG ZUR VERFOLGUNG UND AUFZEICHNUNG DER KIEFERBEWEGUNGEN EINER PERSON UND ENTSPRECHENDES VERFAHREN
APPAREIL DE SUIVI ET D'ENREGISTREMENT DES MOUVEMENTS DE LA MÂCHOIRE D'UNE PERSONNE ET PROCÉDÉ ASSOCIÉ

(30) Priority: 02.05.2019 IT 201900006498
(43) Date of publication of application: 09.03.2022
(73) Proprietor: Rampulla, Giuseppe, 43028 Tizzano Val Parma (PR) (IT); Pighin, Luca, 33080 Zoppola (PN) (IT)
(72) Inventor: FERMI, Enrico, 29122 Piacenza (IT); CROVATO, Diego, 35020 Saonara (PD) (IT); RAMPULLA, Giuseppe, 43028 Tizzano Val Parma (PR) (IT); PIGHIN, Luca, 33080 Zoppola (PN) (IT)
(74) Representative: Fisauli, Beatrice A. M.
(86) International application number: PCT/IB2020/054087
(87) International publication number: WO 2020/222162

(56) References cited:
- WO-A2-2014/004730
- CN-U- 203 914 924
- DE-A1- 3 807 578
- JP-A- 2015 188 485
- US-A1- 2007 280 423
- US-A1- 2019 021 651

## Description

The present invention concerns an apparatus for tracking, reconstructing and recording the movements of a person's jaw during mastication. In particular, the invention concerns an apparatus able to detect and reconstruct the aforesaid movements in a three-dimensional system.

The study of jaw movement and its reconstruction/simulation are particularly useful both for the dentist and for the dental technician. A simple, but rather inaccurate, apparatus used to reconstruct the movements of a jaw is the articulator. The casts of patient's dental arches are positioned in this device, which reproduces the basic opening and closing movement of the jaw. These devices can be used by the dental technician to produce rehabilitation medical devices, such as bridges, mouth splints or the like, which are in turn used by the dentist for the functional or esthetic restoration of a patient's teeth.

With only morphological data of the patient's teeth available, i.e., without information on the occlusal and masticatory function, it is not possible to faithfully replicate the movement of the person's mouth with these articulators. The main limit of apparatus of this kind is in fact the lack of information relating to the kinematics of the masticatory movement of the temporomandibular joint.

With these known apparatus the dentist must therefore subsequently adapt the rehabilitation devices prepared by the dental technician to the patient's actual occlusal condition. This implies an increase in costs and the risk of not obtaining a state-of-the-art rehabilitation device.

For these reasons various methods and related apparatus have been developed to track the movements of a person's jaw, in particular mastication, in order to obtain a digital model thereof that can be used to verify the correct shape and size of the rehabilitation device, for example a dental prosthesis.

Prior art systems are substantially based on three different technologies, namely "motion capture", which uses video cameras and illuminators/projectors, inertial measurements, obtained through the use of accelerometers, gyroscopes or similar devices, and electromagnetic measurements, obtained with magnetic or inductive sensors, etc.

The present invention concerns the first type of apparatus, i.e. those that make use of optical sensors. Among these systems it is possible to further distinguish between systems of "marker based motion capture" type and those of "markerless" type. In the first case, stereo-photogrammetric techniques are used, which allow identification of the three-dimensional position and related movements of markers positioned on specific points of the patient. Within the sphere of analysis of jaw kinematics the aforesaid technique is used, taking as reference the "mandible-maxilla" region in order to obtain jaw kinematics.

An example of prior art methods and related apparatus for registering a part of a patient's body is disclosed in document US 2007/280423 A1. It is disclosed a substance, such as a cream, to be applied to said part of the body. Said substance comprises radiation marking elements. The emitted radiations therefrom can be detected by means of a camera.

Examples of prior art methods and related apparatus for tracking and reconstructing the masticatory movements of a person's jaw are described, for example, in US 2016/0262711 A1, US 5340309 A and WO 2018115576 A1.

However, these prior art systems can be improved. In particular, some of them provide somewhat inaccurate data, for example due to the way in which the markers are fixed to the person's jaw or temporal or maxillary region. Moreover, prior art apparatus are often particularly complex and, besides being very costly, are also complicated to use. Finally, some of the prior art systems are also somewhat invasive and cause discomfort to the patient subjected to analysis.

For these reasons, jaw kinematics analysis applications implemented with optical systems are still relatively limited and not widely developed.

Document WO 2014/004730 A2 discloses an apparatus for tracking and recording the movements of a person's jaw. The apparatus comprises a reference system, an infra-red light projector, a video camera sensitive to infra-red light, a detecting device, a computerized system connected to the projector emitting said infra-red light, and to the video camera, a processing unit and a storage unit. The infrared light is directed from device worn by the person to the detecting device.

However, the apparatus does not yet determine precisely the relative position of the upper and lower jaws.

In this context, the object of the present invention is to solve the limits of the prior art methods and apparatus.

In particular, the object of the invention is to propose an apparatus for tracking the movements of a person's jaw that is simple and practical for an operator to use, but at the same time provides accurate results.

A further object of the present invention is to provide an apparatus of this kind that is simple to use and relatively inexpensive to manufacture.

Another object of the invention is to provide an apparatus for tracking the movements of the jaw that does not cause discomfort to the patient being examined.

These and other objects are achieved with an apparatus that comprises:
- a reference system comprising two parts to be attached to the person's upper and lower dental arches, each part including markers positioned in front of the face;
- an infra-red light projector;
- a video camera sensitive to infra-red light, configured to frame a scene that includes at least said reference system; and
- a computerized system connected to the projector and to the video camera.

According to the invention, the infra-red light projector is placed in front of the person. Said projector is configured to project towards the person, in an area that includes at least the reference system, and preferably also the whole face, infra-red light beams that generate on the illuminated surfaces a pattern of points of light. The video camera is configured to record with a given frequency the projected images of the afore-mentioned pattern of points of light, at least on the reference system. These images can be recorded with the jaw still or moving, for example with a masticatory movement.

The computerized system comprises a processing unit configured to receive the images captured by the infra-red video camera and to calculate, for each image, the distance of at least the points of light that are on the surface of the markers with respect to a pre-determined point with known coordinates. Calculation of the distance takes place by means of a comparison of the image of the actual projected pattern with an image of a reference pattern. Said reference pattern, for example, can be provided by projecting the same light beams on a flat surface, parallel to the plane of the video camera and positioned at a known distance therefrom.

In fact, when the light beams projected by the projector encounter the surfaces of the markers and of the person's face, a variation of the distance between one point and the other is created based on the angle of the surfaces, as the video camera records a two-dimensional projection of the actual (three-dimensional) distribution of said points of light.

Therefore, for each of the points projected it is possible to calculate the distance from the known base point, generally coincident with the sensor of the video camera, triangulating its position with the one it would have in the reference pattern.

The apparatus thus configured is therefore able to directly determine the position in space of the markers, and hence to reconstruct the jaw kinematics in the three dimensions.

Moreover, the measurements to be carried out with this apparatus are not affected by any movements of the person's head while taking the images, as measurement of the distance of the markers is carried out with respect to a known reference system.

The computerized system preferably also includes a storage unit for storing at least the images taken by the video camera and the data calculated by the processing unit.

According to a preferred variant, the video camera is an RGB video camera sensitive to infra-red wavelengths. This makes it possible to record color images that, in the subsequent processing step, better described below, allow more precise recognition of the various elements framed, hence of the surfaces of the markers.

According to an aspect of the invention, each part of the reference system comprises three markers. Said markers preferably have a curved, and more preferably hemispherical, surface. In this way, in the various positions that the marker can take during the movement of the person's jaw or also skull, the surface of the marker always has areas at a different distance (depth) with respect to the video camera. This makes measurement of the distance of the various points of the pattern more accurate and hence gives an accurate reconstruction of the movements in the three dimensions.

According to a preferred embodiment, the markers are mounted on a rigid support and are positioned at the vertices of a triangle. Said triangle is preferably an isosceles triangle and is arranged with its height substantially vertical.

When the reference system is applied to the person, the lower part, i.e. the part attached to the lower dental arch and the jaw, has the apex of the aforesaid triangle facing upwards while the upper part of the reference system, i.e., the part attached to the upper dental arch, has the apex facing downwards, i.e., with said triangle overturned.

This substantially arrow-shaped positioning of the marker allows precise and immediate evaluation of the lateral movements of the mandible with respect to the maxilla.

Advantageously, the supports and the relative markers of the upper and lower part are identical in terms of shape and size so as to optimize both production and procurement costs for users.

According to an aspect of the invention said support comprises a plate made of an opaque material, preferably of a uniform color; typically said support is made of metal or a polymer material.

In this way, the video camera is prevented from recording the part of the person's face immediately behind the markers reducing possible disturbance effects. To this end, the uniform color of the support contributes, making the markers more distinguishable. Preferably, the outer surface of the markers also consists of or is coated with a reflecting material.

The markers have a diameter typically ranging from 5 mm to 20 mm, to offer an area that can be hit by a number of infra-red light beams sufficiently to obtain accurate calculation of the position of the marker.

The distance between the markers of one part, i.e., the length of the sides of the triangle, typically ranges from 30 mm to 60 mm.

According to an aspect of the present invention, said supports are each carried by a rigid supporting element that can be fixed directly to the upper and lower dental arch.

This type of connection makes it possible to reduce measurement errors to a minimum as the markers move substantially rigidly attached to the mandible and the maxilla.

Said supporting element comprises a fastening plate, to be fixed to the teeth by means of an adhesive product, such as resin or the like, and a rod that extends from the fastening plate and carries said support at the end. The rod preferably has a shape or orientation such that when the person's jaw is clamped, the vertices of the two supports of the lower and upper parts are close but do not touch each other.

To avoid change in the person's occlusal function, and hence natural jaw movement, the fastening plates are preferably structured so as to be fixed to the front surface of the teeth.

The method for tracking and recording the movements of a person's jaw according to the invention thus comprises at least the following steps:
- providing at least one infra-red projector and at least one video camera sensitive to infra-red wavelengths;
- providing a reference system comprising two parts, where each part comprises markers;
- fixing the two parts of the reference system to a person's upper and lower dental arches, respectively, in such a way that the markers are positioned in front of the face;
- positioning the person in front of the video camera and the infra-red projector;
- projecting towards the person's face infra-red light beams adapted to form a pattern of points of light, and at the same time to record with a certain frequency images of a scene that includes at least the reference system or the whole face of the person onto which the pattern of points of light is projected;
- processing the images taken with a computerized system configured to compare each image with an image of a reference pattern and to calculate the distance of at least the points of light projected onto the surface of the markers with respect to a pre-determined point with known coordinates.

More in detail, the step of processing the images includes calculating the position of the center of gravity of each marker and tracking their position movements in space. The position of the center of gravity of the various markers, in the preferred variant three in number for each part of the reference system and arranged at the vertices of an isosceles triangle, allows calculation of the center of each part of the reference system.

To improve recognition of the marker with respect to the rest of the scene recorded, the analysis method involves thresholding the brightness levels of the image and simultaneously the color levels.

At this point, subsequent processing of the image allows calculation of the three-dimensional coordinates (X, Y, Z) of the marker, instant by instant, as a function of the acquisition frequency of the video camera.

The method according to the invention involves analysis of the kinematics of the temporomandibular joint both in static position (occlusion) and in movement, for example during mastication.

The kinematic analysis thus obtained is able to provide position, angles and rototranslations of the reference system attached to the jaw and hence comprises all the kinematic information required and sufficient for replication of the movement.

The data provided can be interfaced with a CAD/CAM system and comprise the three coordinates of position and orientation by means of the three Euler angles of both parts of the reference system attached to the person's mandible and maxilla, as well as the position data relating to one part of the reference system with respect to the other (three position and three rotation coordinates).

Further characteristics and details of the invention will be more apparent from the following description, provided by way of non-limiting example, and from the accompanying drawings, wherein:
- Fig. 1 is a perspective view of the reference system of the apparatus according to the invention;
- Fig. 2 is a perspective view of the reference system applied to a person's dental arches;
- Fig. 3 is a schematic view of the apparatus according to the invention in the condition of use.

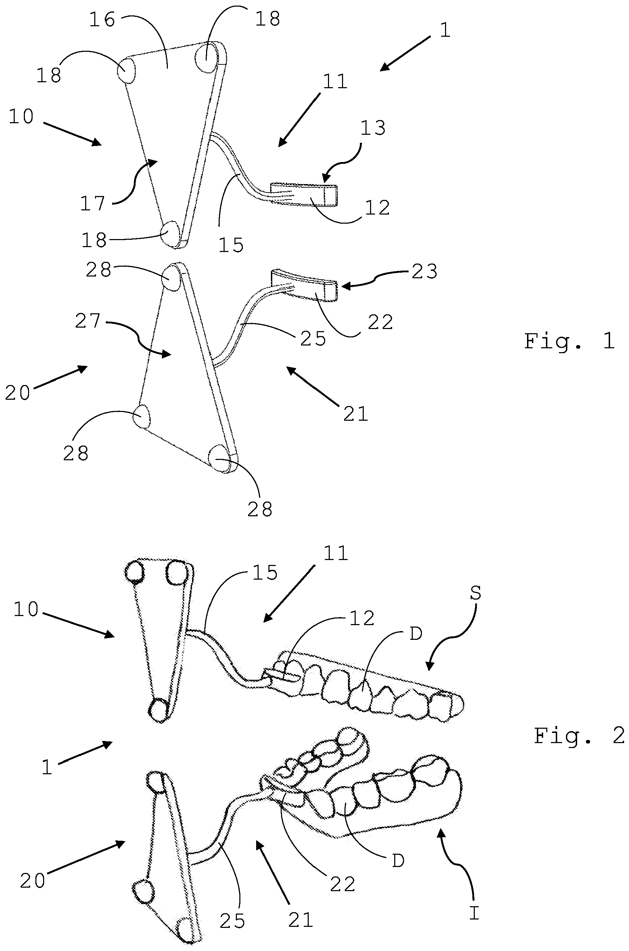

With reference to Fig. 1, this illustrates the reference system 1 of the apparatus according to the invention. The reference system comprises two parts 10, 20 to be attached to the upper and lower dental arch, respectively. Each part comprises a supporting element 11, 21 with a curved fastening plate 12, 22 that has a concave supporting surface 13, 23 adapted to be placed against the front surfaces of the teeth D, as shown in Fig. 2. Fixing of the plate 12, 22 typically takes place by means of gluing, although alternative fixing means, for example of mechanical type, would also be possible.

A curved rod 15, 25, and more precisely curved in a substantially vertical plane, extends from the fastening plate 12, 22. A support 16, 26 comprising a plate in the shape of isosceles triangle is connected to the end of the rod. Preferably, the plate and the rod, which are typically disposable, are connected movably to the support so that the latter can be re-utilized several times for the same person or for different people.

Three hemispherical markers 18, 28 are applied to the vertices of the triangle, on the front surface 17, 27 of the support 16. 26. Said markers have a reflective surface, for example coated with aluminum powder or with a colored reflective paint, preferably of a uniform color.

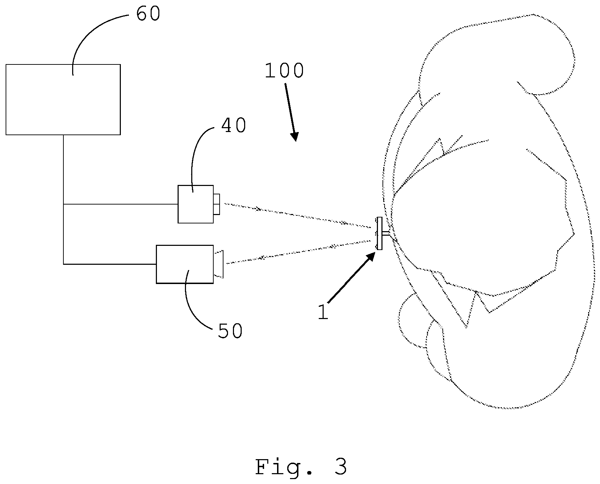

Fig. 3 schematically illustrates the apparatus 100 according to the present invention in an example of use.

In the example in the figure, the reference system 1 is applied to the teeth of a person P whose jaw kinematics require to be tracked. The apparatus 100 further comprises a projector 40 adapted to project infra-red light beams according to a pseudo-random pattern and a video camera 50 of RGB type sensitive to infra-red wavelengths. The video camera 50 and the projector 40 are positioned side by side, facing towards the person's face, as shown in Fig. 3.

The projector 40 and the video camera 50 are connected to a computerized system 60 adapted to receive and process the images taken by the video camera.

The computerized system typically comprises at least a processing unit, a storage unit and preferably also a video interface.

The computerized system is further equipped with a computer program that allows the processing unit to process the images taken to obtain a three-dimensional digital reconstruction of the movement of the person's jaw.

The method implemented through said computerized system involves applying a selective filter to the images to search, in succession, for the greatest brightness and color levels.

Unlike prior art methods commonly used in movement analysis, which are based on calculation of the 2D centers of gravity with subsequent calculation of the three-dimensional coordinates, by means of triangulation, in the present invention three-dimensional data are processed directly.

In detail, the analysis involves the processing of clouds of three-dimensional points of the scene illuminated with the infra-red light beams and recorded by the video camera. The pattern of light beams projected is of a pseudo-random nature and makes it possible to obtain a set of 3D points of coordinates X, Y, Z, which form the hemispherical surfaces of the markers.

The processing method of the images then involves applying a filter to all the 3D points by means of statistical operators to eliminate those that do not satisfy certain criteria.

In particular, the distribution of the distances between the nearby points is calculated and more precisely for each point the average distance between it and all its nearby points is calculated. Assuming that the resulting distribution is of Gaussian type with a mean and standard deviation, all the points whose mean distances are outside a range defined by the global mean of the distances and by the standard deviation are considered as abnormal values and hence removed from the set of data.

Euclidean clustering analysis is applied to the remaining data, i.e., the data are grouped into sub-groups, or clusters, which comprise the points with mathematical properties "similar" to one another, specifically those belonging to the hemispherical surfaces of the markers. This algorithm makes it possible to identify the clusters in which the elements belonging to them are more "similar" to one another with respect to the elements belonging to other sub-groups. The measurement of similarity is a function of the Euclidean distance between the objects and allows the construction of spherical clusters with similar sizes and densities. These spherical clusters correspond to the markers of the reference system.

The subsequent processing is the reconstruction of the effective spherical shape, for example by means of the MLS (Moving Least Squares) technique, which performs smoothing and resampling filtering of the data, to eliminate any discontinuities.

However, the distances calculated and used in the preceding step can, in some cases, be affected by small errors that lead to irregularities in the data that are difficult to eliminate with the statistical method alone. To overcome this, a resampling is preferably carried out to attempt to recreate the missing parts of the surface of the marker by means of polynomial interpolation of higher degree between the surrounding data points.

In the scientific literature, processes for reconstructing surfaces based on polynomial approximation methods (least squares approximation) are frequently described, but not to obtain interpolated data. Therefore, in the analysis method, the MLS Moving Least Squares approximation variant, associated with appropriate weights, is implemented.

At this point, the centers of these hemispheres are calculated by means of an algorithm for estimation of the parameters of the spherical surface and for estimation of the normal directions of each point.

Once the centers of the markers of the two parts of the reference system applied to the person have been defined, the center of the Cartesian coordinate system attached to the lower dental arch and to the upper dental arch is calculated.

The systems are referred with respect to the movement space of the patient and can provide data relating to the movement between them.

With the algorithm implemented in the present invention, measuring as reference parameter a distance between two markers, a precision in the order of units per thousand is obtained.

The present invention is set out in the appended claims.

## Claims

1. An apparatus (100) for tracking and recording the movements of a person's jaw, comprising:
- a reference system (1) comprising two parts (10, 20) to be attached to the person's upper (S) and lower (I) dental arches, respectively, each part including markers (18, 28) positioned in front of the face;
- an infra-red light projector (40);
- a video camera (50) sensitive to infra-red light, configured to frame a scene that includes at least said reference system (1); and
- a computerized system (60) connected to the projector (40) and to the video camera (50);
wherein said projector (40) is configured to project towards the person, in an area that includes at least the reference system (1), infra-red light beams that generate on the illuminated surfaces a pattern of points of light, wherein the video camera (50) is configured to record with a given frequency the projected images of the afore-mentioned pattern of points of light, at least on the markers (18, 28) of the reference system (1), and wherein said computerized system (60) comprises:
- a processing unit configured to receive the images taken by the infra-red video camera and to calculate, for each image, the distance of at least the points of light that are on the surface of the markers (18, 28) with respect to a pre-determined point with known coordinates, by means of a comparison of the image of the projected pattern with an image of a reference pattern;
- a storage unit for storing at least the images taken by the video camera and the data calculated by the processing unit.

2. Apparatus (100) according to claim 1, wherein generation of the infra-red light beams is of a pseudo-random nature.

3. Apparatus (100) according to claim 1 or 2, wherein each part (10, 20) of the reference system comprises three hemispherical markers.

4. Apparatus (100) according to any of the previous claims, wherein the markers (18, 28) have an outer surface consisting of or coated with a reflecting material.

5. Apparatus (100) according to any of the previous claims, wherein the markers (18, 28) are mounted on a rigid support (16, 26) and are positioned at the vertices of an isosceles triangle arranged with its height substantially vertical.

6. Apparatus (100) according to the previous claim, wherein, when the reference system (1) is applied to the person, the lower part (20) has the apex of the triangle turned upwards while the upper part (10) of the reference system (1) has the apex turned downwards.

7. Apparatus (100) according to claim 5 or 6, wherein the supports (16, 26) and relative markers (18, 28) of the upper and lower parts are identical in terms of shape and size.

8. Apparatus (100) according to any of the claims from 5 to 7, wherein said support (16, 26) comprises a plate made of an opaque material or a material of a uniform color.

9. Apparatus according to any of the claims from 5 to 8, wherein said supports (16, 26) are each carried by a rigid supporting element (11, 21) that can be directly fixed to the upper or lower dental arch, respectively.

10. Apparatus according to claim 9, wherein said supporting element (11, 21) comprises a fastening plate (12, 22), to be fixed to the teeth by means of an adhesive product or by mechanical means, and a rod (15, 25) that extends from the fastening plate (12, 22) and carries the support (16, 26) at the end.

11. A method for tracking and recording the movements of a person's jaw, using the apparatus of anyone of claims 1-10, the method comprising at least the following steps:
- fixing the two parts (10, 20) of the reference system (1) to a person's upper and lower dental arches, respectively, in such a way that the markers (18, 28) are positioned in front of the face;
- positioning the person in front of the video camera (50) and the infra-red projector (40);
- projecting towards the person's face infra-red light beams adapted to form a pattern of points of light, and at the same time to record with a certain frequency images of a scene that includes at least the reference system (1) or the whole face of the person onto which the pattern of points of light is projected;
- processing the images taken in real time with the computerized system by comparing each image with an image of a reference pattern and by calculating the distance of at least the points of light projected onto the surface of the markers (18, 28) with respect to a pre-determined point with known coordinates.

## Patentansprüche

1. Eine Vorrichtung (100) zum Verfolgen und Aufzeichnen der Bewegungen des Kiefers einer Person, umfassend:
- ein Bezugssystem (1), umfassend zwei Teile (10, 20), die an den oberen (S), beziehungsweise den unteren (I) Zahnbögen der Person zu befestigen sind, wobei jedes Teil Markierungen (18, 28) enthält, die vor dem Gesicht positioniert werden,
- einen Infrarotlichtprojektor (40);
- eine für Infrarotlicht empfindliche Videokamera (50), die so konfiguriert ist, dass sie eine Szene einfasst, die zumindest das besagte Bezugssystem (1) enthält; und
- ein computergestütztes System (60), das mit dem Projektor (40) und der Videokamera (50) verbunden ist;
wobei der besagte Projektor (40) so konfiguriert ist, dass er auf die Person in einem Bereich, der zumindest das Bezugssystem (1) einschließt, Infrarotlichtstrahlen projiziert, die auf den beleuchteten Oberflächen ein Muster von Lichtpunkten erzeugen, wobei die Videokamera (50) so konfiguriert ist, dass sie mit einer gegebenen Frequenz (60) die projizierten Bilder des zuvor genannten Musters von Lichtpunkten zumindest auf den Markierungen (18, 20) des Bezugssystems (1) aufzeichnet, und wobei das besagte computergestützte System (60) Folgendes umfasst:
- eine Verarbeitungseinheit, die so konfiguriert ist, dass sie die von der Infrarotvideokamera aufgenommenen Bilder empfängt und für jedes Bild den Abstand von mindestens den Lichtpunkten, die sich auf der Oberfläche der Markierungen (18, 28) befinden, in Bezug auf einen vorherbestimmten Punkt mit bekannten Koordinaten berechnet, und zwar durch einen Vergleich des Bildes des projizierten Musters mit einem Bild eines Bezugsmusters;
- eine Speichereinheit zum Speichern zumindest der von der Videokamera aufgenommenen Bilder und der von der Verarbeitungseinheit berechneten Daten.

2. Vorrichtung (100) gemäß Anspruch 1, wobei die Erzeugung der Infrarotlichtstrahlen pseudozufällig ist.

3. Vorrichtung (100) gemäß Anspruch 1 oder 2, wobei jedes Teil (10, 20) des Bezugssystems drei halbkugelförmige Markierungen umfasst.

4. Vorrichtung (100) gemäß einem jeden der vorhergehenden Ansprüche, wobei die Markierungen (18, 28) eine Außenfläche haben, die aus einem reflektierenden Material besteht oder damit beschichtet ist.

5. Vorrichtung (100) gemäß einem jeden der vorhergehenden Ansprüche, wobei die Markierungen (18, 28) auf einer starren Stütze (16, 26) montiert und an den Spitzen eines gleichschenkligen Dreiecks angeordnet sind, dessen Höhe im Wesentlichen vertikal ist.

6. Vorrichtung (100) gemäß dem vorhergehenden Anspruch, wobei beim Anlegen des Bezugssystems (1) an die Person das untere Teil (20) mit der Spitze des Dreiecks nach oben, während das obere Teil (10) des Bezugssystems (1) mit der Spitze nach unten gerichtet ist.

7. Vorrichtung (100) gemäß Anspruch 5 oder 6, wobei die Stützen (16, 26) und die entsprechenden Markierungen (18, 28) der oberen und unteren Teile in Form und Größe identisch sind.

8. Vorrichtung (100) gemäß einem jeden der Ansprüche von 5 bis 7, wobei die besagte Stütze (16, 26) eine Platte aus einem opaken Material oder einem Material mit einer einheitlichen Farbe umfasst.

9. Vorrichtung (100) gemäß einem jeden der Ansprüche von 5 bis 8, wobei die besagten Stützen (16, 26) jeweils von einem starren Stützelement (11, 21) getragen werden, das direkt an dem oberen beziehungsweise unteren Zahnbogen befestigt werden kann.

10. Vorrichtung (100) gemäß Anspruch 9, wobei das besagte Stützelement (11, 21) eine Befestigungsplatte (12, 22) umfasst, die mittels eines Klebeprodukts oder durch mechanische Mittel an den Zähnen zu befestigen ist, und eine Stange (15, 25), die sich von der Befestigungsplatte (12, 22) aus erstreckt und die Stütze (16, 26) am Ende trägt.

11. Ein Verfahren zum Verfolgen und Aufzeichnen der Bewegungen des Kiefers einer Person unter Verwendung der Vorrichtung nach einem jeden der Ansprüche 1-10, wobei das Verfahren mindestens folgende Schritte umfasst:
- Befestigung der beiden Teile (10, 20) des Bezugssystems (1) an den oberen beziehungsweise unteren Zahnbögen einer Person, so dass die Markierungen (18, 28) vor dem Gesicht positioniert sind,
- Positionierung der Person vor der Videokamera (50) u dem Infrarotprojektor (40);
- Projizieren von Infrarotlichtstrahlen auf das Gesicht der Person, die geeignet sind, ein Muster von Lichtpunkten zu bilden und gleichzeitig mit einer bestimmten Frequenz Bilder einer Szene aufzuzeichnen, die zumindest das Bezugssystem (1) oder das gesamte Gesicht der Person einschließt, auf das das Muster von Lichtpunkten projiziert wird;
- Verarbeitung der mit dem computergestützten System in Echtzeit aufgenommenen Bilder durch Vergleich jedes Bildes mit einem Bild eines Bezugsmusters und durch Berechnung des Abstands mindestens der auf die Oberfläche der Markierungen (18, 28) projizierten Lichtpunkte in Bezug auf einen vorherbestimmten Punkt mit bekannten Koordinaten.

## Revendications

1. Appareil (100) pour suivre et enregistrer les mouvements de la mâchoire d'une personne, comprenant :
- un système de référence (1) comprenant deux parties (10, 20) à fixer sur les arcades dentaires supérieure (S) et inférieure (I) de la personne, respectivement, chaque partie comprenant des marqueurs (18, 28) positionnés devant le visage ;
- un projecteur de lumière infrarouge (40) ;
- une caméra vidéo (50) sensible à la lumière infrarouge, configurée pour cadrer une scène qui comprend au moins ledit système de référence (1) ; et
- un système informatisé (60) connecté au projecteur (40) et à la caméra vidéo (50) ;
où ledit projecteur (40) est configuré pour projeter vers la personne, dans une zone qui inclut au moins le système de référence (1), des faisceaux de lumière infrarouge qui génèrent sur les surfaces éclairées un motif de points lumineux, où la caméra vidéo (50) est configurée pour enregistrer avec une fréquence donnée les images projetées du motif de points lumineux susmentionné, au moins sur les marqueurs (18, 28) du système de référence (1), et où ledit système informatisé (60) comprend :
- une unité de traitement configurée pour recevoir les images prises par la caméra vidéo infrarouge et pour calculer, pour chaque image, la distance des points lumineux au minimum qui se trouvent sur la surface des marqueurs (18, 28) par rapport à un point prédéterminé dont les coordonnées sont connues, au moyen d'une comparaison de l'image du motif projeté avec une image d'un motif de référence ;
- une unité de stockage pour stocker au moins les images prises par la caméra vidéo et les données calculées par l'unité de traitement.

2. Appareil (100) selon la revendication 1, où la génération des faisceaux lumineux infrarouges est de nature pseudo-aléatoire.

3. Appareil 100) selon la revendication 1 ou 2, où chaque partie (10, 20) du système de référence comprend trois marqueurs hémisphériques.

4. Appareil 100) selon l'une des revendications précédentes, où les marqueurs (18, 28) ont une surface extérieure constituée ou revêtue d'un matériau réfléchissant.

5. Appareil (100) selon l'une des revendications précédentes, dans lequel les marqueurs (18, 28) sont montés sur un support rigide (16, 26) et sont positionnés aux sommets d'un triangle isocèle disposé avec sa hauteur sensiblement verticale.

6. Appareil (100) selon la revendication précédente, dans lequel, lorsque le système de référence (1) est appliqué sur la personne, la partie inférieure (20) présente le sommet du triangle tourné vers le haut tandis que la partie supérieure (10) du système de référence (1) présente le sommet tourné vers le bas.

7. Appareil (100) selon la revendication 5 ou 6, où les supports (16, 26) et les repères relatifs (18, 28) des parties supérieure et inférieure sont identiques en termes de forme et de taille.

8. Appareil (100) selon l'une des revendications de 5 à 7, dans lequel ledit support (16, 26) comprend une plaque en matériau opaque ou de couleur uniforme.

9. Appareil selon l'une des revendications de 5 à 8, dans lequel lesdits supports (16, 26) sont chacun portés par un élément de support rigide (11, 21) qui peut être directement fixé à l'arcade dentaire supérieure ou inférieure, respectivement.

10. Appareil selon la revendication 9, où ledit élément de support (11, 21) comprend une plaque de fixation (12, 22), destinée à être fixée sur les dents au moyen d'un produit adhésif ou par des moyens mécaniques, et une tige (15, 25) qui s'étend à partir de la plaque de fixation (12, 22) et porte le support (16, 26) à son extrémité.

11. Méthode de suivi et d'enregistrement des mouvements de la mâchoire d'une personne à l'aide de l'appareil de l'une des revendications 1 à 10, la méthode comprenant au moins les étapes suivantes :
- fixer les deux parties (10, 20) du système de référence (1) aux arcades dentaires supérieure et inférieure d'une personne, respectivement, de manière à ce que les marqueurs (18, 28) soient positionnés devant le visage ;
- placer la personne devant la caméra vidéo (50) et le projecteur infrarouge (40) ;
- projeter vers le visage de la personne des faisceaux lumineux infrarouges adaptés pour former un motif de points lumineux, et en même temps pour enregistrer avec une certaine fréquence des images d'une scène qui comprend au moins le système de référence (1) ou le visage entier de la personne sur lequel le motif de points lumineux est projeté ;
- traiter les images prises en temps réel avec le système informatisé en comparant chaque image avec une image d'un motif de référence et en calculant la distance d'au moins les points lumineux projetés sur la surface des marqueurs (18, 28) par rapport à un point prédéterminé dont les coordonnées sont connues.
